(19) Europäisches Patentamt

European Patent Office

Office européen des brevets

(11) Publication number: **0 332 346**
A2

# EUROPEAN PATENT APPLICATION

(21) Application number: 89302100.6

(22) Date of filing: 02.03.89

(51) Int. Cl.⁴: **A61K 45/06** , **A61K 37/54** ,
//(A61K45/06,31:445),
(A61K37/54,31:445)

(30) Priority: 03.03.88 GB 8805077

(43) Date of publication of application:
13.09.89 Bulletin 89/37

(84) Designated Contracting States:
AT BE CH DE FR GB IT LI NL SE

(71) Applicant: GLAXO GROUP LIMITED
Clarges House 6-12 Clarges Street
London W1Y 8DH(GB)

(72) Inventor: Humphrey, Patrick Paul Anthony
New Sands End Harlton Road
Little Eversden Cambridgeshire(GB)
Inventor: Johnson, Malcolm
10 Greenford Close
Orwell Cambridgeshire(GB)

(74) Representative: Brewer, Christopher Laurence
Glaxo Holdings p.l.c. 63 Graham Street
London N1 8JZ(GB)

(54) Pharmaceutical combinations containing a piperidinylcyclopentylheptenoic acid derivative.

(57) The use is described of both (i) [1R-[1α(Z),2β,3β,5α]]-(+)-7-[5-[[1,1'-biphenyl)-4-yl]methoxy]-3-hydroxy-2-(1-piperidinyl) cyclopentyl]-4-heptenoic acid or a physiologically acceptable salt, solvate or cyclodextrin complex thereof and (ii) a thrombolytic agent in the therapy or prophylaxis of thrombotic disorders.
Pharmaceutical compositions containing both (i) and (ii) are also described.

EP 0 332 346 A2

## PHARMACEUTICAL COMBINATIONS CONTAINING A PIPERIDINYLCYCLOPENTYLHEPTENOIC ACID DE-RIVATIVE

The present invention relates to a combination of a thrombolytic agent with $[1R-[1\alpha(Z),2\beta,3\beta,5\alpha]]-(+)-7-[5-[[(1,1'-biphenyl)-4-yl]methoxy]-3-hydroxy-2-(1-piperidinyl)cyclopentyl]-4-heptenoic$ acid (hereinafter referred to as Compound A) or a physiologically acceptable salt, solvate or cyclodextrin complex thereof, to pharmaceutical formulations containing them, and to their use in human or veterinary medicine.

Blood clots are composed of fibrin which forms from its soluble precursor fibrinogen under the action of the enzyme thrombin. Mammalian plasma contains an enzymatic system capable of dissolving the fibrin in blood clots. In cases where the thrombolytic potential of the body is insufficient to remove intravascular thrombi it is often necessary to use exogenously administered thrombolytic agents. However, although thrombolytic agents have been shown in human studies to rapidly dissolve blood clots, it has also been demonstrated that after administration the obstruction is not resolved or the obstruction re-occurs in a significant proportion of patients.

Our UK Patent Specification 2097397 describes inter alia Compound A which is a potent thromboxane receptor blocker. We have stated therein that such compounds inhibit thromboxane $A_2$ and endoperoxide mediated aggregration of blood platelets and contraction of vascular smooth muscle and are thus of particular interest as anti-thrombotic agents.

We have now found that it is beneficial to co-administer a thrombolytic agent and Compound A or a physiologically acceptable salt, solvate or cyclodextrin complex thereof. Compound A or a physiologically acceptable salt, solvate or cyclodextrin complex thereof and a thrombolytic agent in combination therapy is therefore of interest for use in human and veterinary medicine, more particularly for use in the treatment of prophylaxis of thrombotic disorders. The benefit of co-administration of the two agents derives from the enhancement of therapeutic effect when both agents are used in the treatment or prophylaxis of such conditions. Particular examples of thrombotic disorders are known in the art but include myocardial infarction, deep vein thrombosis, retinal vein thrombosis, pulmonary embolism, stroke and peripheral vascular disease.

Thus, according to one aspect of the invention, we provide a method of treating thrombotic disorders in human or animal subjects which comprises administering to the patient effective amounts of a thrombolytic agent and Compound A or a physiologically acceptable salt, solvate or cyclodextrin complex thereof.

It will be appreciated that the thrombolytic agent and Compound A or a physiologically acceptable salt, solvate or cyclodextrin complex thereof may be administered to the patient either simultaneously, sequentially or in combination. If there is sequential administration, the delay in administering the second of the active ingredients should not be such as to lose the benefit of a potentiated thrombolytic or anti-thrombotic effect of the combination of the active ingredients.

The thrombolytic agent and Compound A or a physiologically acceptable salt, solvate or cyclodextrin complex thereof will preferably be administered to the patient simultaneously. However, if simultaneous administration is not practicable, the active agents are preferably administered sequentially, the administration of one agent immediately followed by administration of the other agent. When the two active agents are administered in this way Compound A or a physiologically acceptable salt, solvate or cyclodextrin complex thereof is conveniently given intravenously.

It may prove desirable for the patient to initially receive a loading dose of Compound A or a physiologically acceptable salt, solvate or cyclodextrin complex thereof followed by administration of both active agent as described just above.

It may also prove desirable to continue treatment with Compound A or a physiologically acceptable salt, solvate or cyclodextrin complex thereof to maximise inhibition of post-therapy reocclusion. Thus, for example, oral maintenance therapy can be continued, e.g. for six months.

In another aspect of the invention, therefore, we provide a thrombolytic agent and Compound A or a physiologically acceptable salt, solvate or cyclodextrin complex thereof in the presence of each other in the patient for use in human or veterinary medicine, more particularly for use in the treatment or prophylaxis of thrombotic disorders.

In a further aspect of the invention we provide a combination of a thrombolytic agent and Compound A or a physiologically acceptable salt, solvate or cyclodextrin complex thereof.

In a yet further aspect of the invention we provide a combination of a thrombolytic agent and Compound A or a physiologically acceptable salt, solvate or cyclodextrin complex thereof for use in human or veterinary medicine, more particularly for use in the treatment or prophylaxis of thrombotic disorders.

In another aspect of the invention we provide the use of a thrombolytic agent and Compound A or a

2

physiologically acceptable salt, solvate or cyclodextrin complex thereof in the preparation of a medicament for the treatment of prophylaxis of thrombotic disorders in the human or animal body.

In using a thrombolytic agent and Compound A or a physiologically acceptable salt, solvate or cyclodextrin complex thereof according to the present invention, it is preferable to employ them in the form of pharmaceutical formulations which may be in separate formulations or in a single combined formulation. However, in the latter formulation both active ingredients must of course be stable and mutually compatable in the particular formulation employed.

The present invention therefore also provides a pharmaceutical composition which comprises a thrombolytic agent and Compound A or a physiologically acceptable salt, solvate or cyclodextrin complex thereof together, where desirable, with one or more pharmaceutical carriers or excipients.

In another aspect of the invention we provide a pharmaceutical composition comprising a thrombolytic agent and Compound A or a physiologically acceptable salt, solvate or cyclodextrin complex thereof together, where desirable, with one or more pharmaceutical carriers or excipient for use in human or veterinary medicine, more particularly for use in the treatment or prophylaxis or thrombotic disorders.

The single combined composition may be prepared by admixture of the active ingredients together, where desirable, with one or more pharmaceutical carriers or excipients. Thus, in another aspect of the invention we provide a process for the preparation of a pharmaceutical composition which comprises mixing a thrombolytic agent and Compound A or a physiologically acceptable salt, solvate or cyclodextrin complex thereof together, where desirable, with one or more pharmaceutical carriers or excipients.

When the thrombolytic agent and Compound A or a physiologically acceptable salt, solvate or cyclodextrin complex thereof are to be used as a single combined composition, the composition will generally be administered parenterally (e.g. intramuscularly, subcutaneously, intraperitoneally or intravenously), and either by infusion or by bolus injection. Intravenous administration is the preferred route of administration for a single combined composition, the composition being preferably administered by infusion.

Parenteral formulations containing the thrombolytic agent and Compound A or a physiologically acceptable salt, solvate or cyclodextrin complex thereof in a single combined composition may be prepared in a similar manner to the preparation of formulations suitable for the thrombolytic agent or Compound A or a physiologically acceptable salt, solvate or cyclodextrin complex thereof per se. The parenteral formulations may be presented in the form of suspensions, solutions or emulsions in oil or aqueous vehicles which may optionally contain formulatory agents such as suspending, stabilising and/or dispersing agents. Alternatively, the active ingredients may be in powder form for reconstitution before use with a suitable vehicle, e.g. sterile pyrogen-free water. Formulations for injections may be presented in unit dosage form in ampoules, or in a multidose container with an added preservative, or in a suitable container for infusion.

As stated hereinbefore, the thrombolytic agent and Compound A or a physiologically acceptable salt, solvate or cyclodextrin complex thereof may be administered as two separate compositions. The thrombolytic agent will generally be in a form suitable for parenteral administration. Compound A or a physiologically acceptable salt, solvate or cyclodextrin complex thereof may be used in various pharmaceutical formulations, especially for oral or parenteral administration.

It may be convenient to present the thrombolytic agent and Compound A or a physiologically acceptable salt, solvate or cyclodextrin complex thereof as a two container pack, one container containing the thrombolytic agent and the other containing Compound A or a physiologically acceptable salt, solvate or cyclodextrin complex thereof. The compounds may then be admixed immediately before administration or, if desired, may be administered separately either simultaneously or sequentially.

The invention also provides a thrombolytic agent and Compound A or a physiologically acceptable salt, solvate or cyclodextrin complex thereof and compositions containing them, in association with instructions for their use together in human or veterinary medicine, more particularly for their use together in the treatment or prophylaxis of thrombotic disorders.

It is to be understood that when the active agents are administered simultaneously, either in a single combined composition or separately, the appropriate composition will comprise an amount of Compound A or a physiologically acceptable salt, solvate or cyclodextrin complex thereof effective to inhibit platelet aggregation at the site of the occlusion during the period of thrombolytic therapy and an amount of the thrombolytic agent effective to lyse a target thrombotic occlusion.

It will be appreciated that any thrombolytic agent compatable with Compound A or a physiologically acceptable salt, solvate or cyclodextrin complex thereof may be used according to the present invention. Important thrombolytic agents for use according to the invention include the enzymes, plasminogen activators. Examples of suitable plasminogen activators include "kinases" such as streptokinase, streptokinase/plasminogen complex, urokinase and prourokinase, tissue plasminogen activator (hereinafter referred to as 't-PA') and the like.

It is to be understood that the term "thrombolytic agent" as used herein is intended to also generally cover agents which, when administered, cause an increase in fibrinolysis, for example through endogenous t-PA release. Such agents include vasopressin analogues, pentosan polysulphate and Protein C. Vasopressin analogues are generally well-known in the art and suitable vasopressin analogues for use according to the present invention include analogues described by A. M. A. Gader et. al. in The Lancet 1973, Volume 2, 1417-1418. Pentosan polysulphate has been reported in the literature, for example in Thrombosis and Haemostasis 1985, 54 (4), 833-837 (N. A. Marsh et. al.), and Protein C has been described in numerous publications including Thrombosis Research 41, 483-488 (1986) by C. Sharon et. al.

The plasminogen activators streptokinase and urokinase are both commercially available for thrombolytic therapy and parenteral pharmaceutical formulations containing streptokinase or urokinase are well-known in the art. A particularly convenient urokinase for use according to the present invention is now commercially available as 'Breokinase' or 'Alphakinase'.

The term 'streptokinase/plasminogen complex' means herein any binary complex of streptokinase and plasminogen including blocked forms of the streptokinase/plasminogen complex as described, for example, in EP-A-009879 and EP-A-0028489. Particularly important streptokinase/plasminogen complexes for use according to the present invention include binary complexes between streptokinase and plasminogen wherein the catalytic site essential for fibrinolytic activity is blocked by an acyl group. A particularly convenient streptokinase/plasminogen complex for use according to the present invention is now commercially available as 'Eminase'. The streptokinase/plasminogen complexes and parenteral pharmaceutical formulations containing them may be prepared according to methods described in the aforementioned European patent specifications.

The term 'prourokinase' means herein any single chain polypeptide substantially corresponding to the single chain precursor of urokinase SCUPA (single chain urinary plasminogen activator). Important single chain polypeptides for use according to the present invention include those which are resistant to conversion to high or low molecular weight urokinase. Examples of suitable polypeptides are described in EP-A-139447, EP-A-200451, EP-A-236040 and EP-A-247674. The prourokinase polypeptides and parenteral pharmaceutical formulations containing them may be prepared according to the methods described in the aforementioned European patent specifications or in EP-A-232544.

The term 't-PA' means herein any bioactive protein substantially corresponding to mammalian, and especially human, t-PA and includes forms with or without glycosylation. The t-PA may be one- or two-chain t-PA or a mixture thereof, although preferably the t-PA is in a single chain form for use according to the present invention. A number of t-PAs are known in the art, for example, those described in EP-A-41766, EP-A-93619, EP-A-112122, EP-A-113319, EP-A-117059, EP-A-117060, EP-A-173552, EP-A-174835, EP-A-178105, EP-A-225117, EP-A-225286, WO 86/01538, WO 86/05514, WO 86/05807, GB-A-2173804, GB-A-2176702, GB-A-2176703, GB-A-2184354 and GB-A-2189249. A particularly convenient t-PA for use according to the present invention is the human t-PA product now commercially available as 'Activase'. The t-PA and parenteral pharmaceutical formulations containing the t-PA may be prepared according to methods described in the aforementioned European and British patent specifications or in EP-A-123304, EP-A-143081, EP-A-156169, EP-A-211592, EP-A-217379, EP-A-218112, EP-A-232544, or WO 86/01104.

Particularly suitable thrombolytic agents for use according to the present invention are the plasminogen activators referred to above as "kinases" (i.e. plasminogen activators such as streptokinase, streptokinase/plasminogen complex, urokinase and prourokinase). Streptokinase and acylated streptokinase/plasminogen complexes such as 'Eminase' are particularly suitable for use according to the present invention.

Suitable salts of Compound A include acid addition salts derived from inorganic and organic acids such as hydrochlorides, hyrobromides, sulphates, phosphates, maleates, tartrates, citrates, benzoates, 2-chlorobenzoates, p-toluenesulphonate, methanesulphonates, salicylates, fumarates, lactates, hydroxynaphthalenecarboxylates (e.g. 1-hydroxy- or 3-hydroxy-2-naphthalenecarboxylates) or furoates, or salts with suitable bases such as alkali metal (e.g. sodium and potassium), alkaline earth metal (e.g. calcium or magnesium), ammonium and substituted ammonium (e.g. dimethylammonium, triethylammonium, 2-hydroxyethyldimethylammonium piperazinium, N,N-dimethylpiperazinium, piperidinium, ethlenediaminium and choline) salts. A preferred salt of Compound A is the hydrochloride salt. Compound A and physiologically acceptable salts and solvates thereof and pharmaceutical formulations containing them are described in GB-B-2097397 and GB-B-2127406.

When Compound A is used according to the present invention in the form of a cyclodextrin complex, the complex conveniently contains a molar ratio of Compound A with cyclodextrin within the range 1:1 to 1:3.

4

The term "cyclodextrin" means herein as unsubstituted or substituted $\alpha$-, $\beta$- or $\gamma$-cyclodextrin (or a hydrate thereof) or a mixture of two or three of them. Examples of suitable substituted cyclodextrins include sulphur-containing cyclodextrins, nitrogen-containing cyclodextrins, alkylated (e.g. methylated) cyclodextrins such as mono-, di or trimethylated derivatives of a cyclodextrin (e.g. of $\beta$-cyclodextrin) and hydroxyalkyl (e.g. hydroxypropyl) cyclodextrins such as hydroxypropyl $\beta$-cyclodextrin and acylated derivatives thereof. Hydroxyalkyl (e.g. hydroxypropyl) cyclodextrins such as hydroxypropyl $\beta$-cyclodextrin have been found to be particularly suitable.

A particularly preferred cyclodextrin complex of Compound A is the $\beta$-cyclodextrin complex in which the molar ratio of Compound A with $\beta$-cyclodextrin is about 1:1.

Cyclodextrin complexes of Compound A and pharmaceutical formulations containing them are described in our co-pending British Patent Application No. 8829793. The cyclodextrin complexes may be prepared by mixing Compound A or the hydrochloride salt thereof with the cyclodextrin in a suitable solvent such as water or an organic solvent which is miscible with water (e.g. an alcohol such as methanol). The reaction may take place at any temperature in the range from $0^{\circ}$ to $80^{\circ}$ C. However, the mixture is preferably kept at around room temperature and the desired complex obtained by concentrating the mixture under reduced pressure or by allowing the mixture to cool. The mixing ratio of organic solvent with water may be suitably varied according to the solubilities of the starting materials and products. Preferably 1 to 4 moles of cyclodextrin are used for each mole of Compound A or its hydrochloride salt.

Pharmaceutical formulations containing a cyclodextrin complex of Compound A may be prepared in conventional manner by mixing the complex with one or more pharmaceutical carriers or excipients, for example, according to the general methods described in GB-B-2097397 and GB-B-2127406.

When Compound A or a physiologically acceptable salt, solvate or cyclodextrin complex is to be administered as an aqueous formulation for, in particular, parenteral (e.g. intravenous) use, the composition may be prepared according to the general methods described in GB-B-2097397 and GB-B-2127406. Alternatively, the aqueous compositions may be prepared by mixing Compound A or, more preferably, the hydrochloride salt thereof with cyclodextrin together with one or more pharmaceutical carriers or excipients, for example as described in the Examples hereinafter. Preferably, Compound A or its hydrochloride salt as dissolved in water and the remaining constituents are added thereto.

The molar ratio of Compound A or its hydrochloride salt with cyclodextrin in the aqueous composition is conveniently within the range 1:1 to 1:3.

Preferably, the aqueous formulations comprises the hydrochloride salt of Compound A and $\beta$-cyclodextrin (or a hydrate thereof) at about physiological pH wherein the formulations contains at least about 1.2 moles of $\beta$-cyclodextrin (e.g. 1.2 to 2 moles) for every one mole of the hydrochloride salt of Compound A. Preferably the molar ratio will be about 1:1.4.

The precise dose of the thrombolytic agent and Compound A or a physiologically acceptable salt, solvate or cyclodextrin complex thereof to be administered will, of course, depend on a number of factors including, for example, the age and weight of the patient, the specific condition requiring treatment and its severity and the route of administration. An effective oral dose, however, in the case of Compound A or a physiologically acceptable salt, solvate or cyclodextrin complex thereof is likely to be in the range from 0.05 to 20 mg/kg body weight of patient per day, preferably in the range from 0.05 to 5 mg/kg per day.

The concentration of Compound A or the hydrochloride salt thereof in the aforementioned aqueous formulations suitable for parenteral administration, in particular for administration by injection (eg intravenously), is conveniently within the range 0.1-10mg/ml, e.g. 0.1-5mg/ml, expressed as the free base. Preferably, the concentration is 1mg/ml expressed as the free base when the aqueous formulation is administered by intravenous injection. If desired, a higher concentration may be used and the solution may be diluted prior to use with, for example, an isotonic saline solution or dextrose or mannitol solution. Conveniently, solutions suitable for injection are presented in an appropriate dose volume (eg 1 - 100 ml). Dilutions suitable for continuous infusion may have a concentration of Compound A or its hydrochloride salt of 0.01 - 0.2mg/ml expressed as the free base. The solution for continuous infusion may be presented in this form, for example in packs of 50-100ml, or may be presented in more concentrated forms for subsequent dilution before use with, for example, an isotonic saline solution or dextrose or mannitol solution. Alternatively, small volumes of a more concentrated solution (eg 0,1 - 5 mg/ml) may be utilised for continuous infusion conveniently administered at a rate of 0.5 to 9.9ml/h.

The aforementioned aqueous formulations may also be adapted for oral administration (e.g. as a capsule, syrup or solution). The preparation of suitable formulations for oral use will be within the knowledge of persons skilled in the art and may generally follow the procedures described in GB-B-2097397, GB-B-2127406 and in the Examples hereinafter.

Dosages and dosage rates in the case of the thrombolytic agents will generally correspond with

5

dosages and dosage rates one would use if administering the thrombolytic agent alone to treat the thrombotic disorder. In the case of streptokinase and urokinase effective dose regimes for a variety of thrombotic disorders are now well-established. In the case of a streptokinase/plasminogen complex a patient with a medium-sized thrombus will generally receive a daily dose of from 0.10 to 1.0 mg/kg body weight of patient either by injection in up to eight doses or by infusion. Dosages and dosage rates as described for urokinase are generally equally satisfactory for a prourokinase. Typically a protease-resistant prourokinase will be infused intravenously at a rate usually greater than about 4000 IU/kg/hr to achieve perfusion of an occluded artery or vein. In the case of t-PA an effective dose is likely to be in the range from 150,000 to 450,000 IU/kg/hr, although the dosage is likely to be less for some thrombotic conditions, such as deep vein thrombosis and acute stroke or for maintaining patency of the already reperfused coronary artery. In these situations, an effective amount of t-PA will generally be in the range from 7,000 to 36,000 IU/kg/hr.

The following examples are provided in illustration of the present invention and should not be construed in any way as constituting a limitation thereof.

Pharmaceutical examples of parenteral injections/infusions comprising the hydrochloride salt of Compound A

| (i) | Hydrochloride salt of Compound A equivalent to 50 mg base | | | |
|---|---|---|---|---|
| | β-Cyclodextrin hydrate | 143mg | 166mg | 238mg |
| | Sodium hydroxide solution | to pH7 | to pH7 | to pH7 |
| | Water suitable for injection | to 50ml | to 50ml | to 50ml |

The hydrochloride salt of Compound A was dissolved in 35ml water suitable for injection and the β-cyclodextrin was added. This solution was titrated to pH7 with 0.02M sodium hydroxide solution and then adjusted to volume with water suitable for injection.

The solution may then be sterilised by filtration and filled into vials or ampoules.

| (ii) | Hydrochloride salt of Compound A equivalent to 50mg base | |
|---|---|---|
| | β-Cyclodextrin hydrate | 166mg |
| | Sodium chloride | 450mg |
| | pH7.0 phosphate buffer | 2.5ml |
| | Sodium hydroxide solution | to pH7 |
| | Water suitable for injection | to 50ml |

The hydrochloride salt of Compound A was dissolved in approximately 25ml water suitable for injection. The β-cyclodextrin was dissolved therein and the resulting solution was titrated to pH6 with 0.02M sodium hydroxide solution and the phosphate buffer added. The sodium chloride was added to the solution and the pH adjusted to pH7 with sodium hydroxide. The solution was made up to volume with water suitable for injection. A sample of this solution was filled into a glass vial which was sealed with a rubber plug and metal overseal. This was then autoclaved.

| (iii) | Hydrochloride salt of Compound A equivalent to 50 mg base | |
|---|---|---|
| | Hydroxypropyl-β-cyclodextrin | 170mg |
| | Mannitol | 2.5g |
| | pH 6.0 phosphate buffer | 5.0ml |
| | Sodium hydroxide solution | to pH 6 |
| | Water suitable for injection | to 50ml |

The hydrochloride salt of Compound A was dissolved in approximately 25ml water suitable for injection and the hydroxypropyl-β-cyclodextrin was added. The mannitol was then added and the solution titrated to pH 6 with 0.02M sodium hydroxide solution. The phosphate buffer solution was added and the solution adjusted to volume with water suitable for injection. The solution was then filtered and filled into glass vials which were sealed with rubber plugs and metal overseals. These were then autoclaved.

| (iv) | Hydrochloride salt of Compound A equivalent to 50mg base | |
|---|---|---|
| | β-Cyclodextrin hydrate | 166mg |
| | Mannitol | 2.5g |
| | Sodium acid phosphate | 46mg |
| | Disodium phosphate, anhydrous | 5mg |
| | Sodium hydroxide solution | to pH 6 |
| | Water suitable for injection | to 50ml |

The hydrochloride salt of Compound A was dissolved in approximately 25ml water suitable for injection. The β-cyclodextrin and mannitol were dissolved therein and the solution titrated to pH 6 with 0.02M sodium hydroxide solution. The sodium acid phosphate and anhydrous disodium phosphate were dissolved in water suitable for injection. This solution was added to the bulk solution which was made up to volume with water suitable for injection. The solution was filtered and filled into glass ampoules which were sealed and then autoclaved.

| (v) | | Cyclodextrin | | | |
|---|---|---|---|---|---|
| | | | | Mixture | |
| | | α | γ | β + γ | |
| Hydrochloride salt of Compound A equivalent to 50mg base Cyclodextrin | | 143mg | 190mg | 119mg | 136mg |
| Mannitol | | 2.5g | 2.5g | 2.5g | |
| pH 6.0 Phosphate buffer | | 5.0ml | 5.0ml | 5.0ml | |
| Sodium hydroxide solution | | to pH 6 | to pH 6 | to pH 6 | |
| Water suitable for injection | | to 50ml | to 50ml | to 50ml | |

The hydrochloride salt of Compound A was dissolved in approximately 25ml water suitable for injection and the cyclodextrin(s) was (were) added. The mannitol was then added and the solution titrated to pH 6 with 0.02M sodium hydroxide solution. The phosphate buffer solution was added and the solution was adjusted to volume with water suitable for injection. The solution was then filtered and filled into glass vials which were sealed with rubber plugs and metal overseals.

Pharmaceutical example of an oral syrup comprising the hydrochloride salt of Compound A

| Hydrochloride salt of Compound A equivalent to 2.5mg base | |
|---|---|
| β-cyclodextrin hydrate | 9mg |
| Citric acid | to pH 4.5 |
| Methyl hydroxybenzoate sodium | 5mg |
| Propyl hydroxylbenzoate sodium | 2mg |
| Liquid orange flavour | qs |
| Sucrose | 3.25g |
| Purified Water | to 5.0ml |

Dissolve the sucrose in a minimum quantity of water. Add the hydrochloride salt of Compound A and then the β-cyclodextrin with stirring; adjust the pH to 4.5 with citric acid. With continued stirring add a solution of the hydroxybenzoates and lastly the flavour. Adjust almost to volume with water and stir. Check the pH and adjust to 4.5 with citric acid if necessary. Make up to volume with water.

**Claims**

1. The use of (i) [1R-[1α(Z),2β,3β,5α]]-(+)-7-[5-[[(1,1'-biphenyl)-4-yl]methoxy]-3-hydroxy-2-(1-piperidinyl)cyclopentyl]-4-heptenoic acid or a physiologically acceptable salt, solvate or cyclodextrin complex thereof and (ii) a thrombolytic agent in the manufacture of medicaments for the use either separately or in combination of (i) and (ii) and in the presence of each other in the human body for the therapy or prophylaxis of thrombotic disorders.

2. The use according to Claim 1 in which the compounds (i) and (ii) are presented as separate compositions for said use.

3. A pharmaceutical composition which comprises both a compound (i) and a compound (ii) as defined in Claim 1.

4. The use or a composition according to any preceding claim in which compound (i) is in the form of its hydrochloride salt.

5. The use or a composition according to any preceding claim in which compound (ii) is a kinase plasminogen activator.

6. The use or a composition according to any preceding claim in which compound (ii) is streptokinase.

7. The use or a composition according to any of Claims 1 to 5, in which compound (ii) is an acylated streptokinase/plasminogen complex.

8. The use according to any of Claims 2, 4, 5, 6 and 7 in which the hydrochloride salt of compound (i) is in a form suitable for oral or parenteral administration.

9. The use according to any of Claims 2, 4, 5, 6, 7 and 8 in which the hydrochloride salt of compound (i) is in a form suitable for intravenous administration.

10. The two-container pack for use in the therapy or prophylaxis of thrombotic disorders in humans, one of the containers containing a compound (i) and the other containing a compound (ii) as defined in the preceding claims.

11. A composition according to any of Claims 3 to 7, or a pack according to Claim 10 or (i) or (ii) as defined in Claim 1 in association with instructions for the use of both (i) and (ii) in the therapy or prophylaxis of thrombotic disorders in humans.

12. The use of both compounds (i) and (ii) as defined in the preceding claims in the presence of each other in the human body in therapy.

13. The use of both compounds (i) and (ii) as defined in the preceding claims in the presence of each other in the human body in the therapy or prophylaxis of thrombotic disorders.